# EUROPEAN PATENT APPLICATION

(11) **EP 4 685 232 A1**
(43) Date of publication of application: **28.01.2026**
(21) Application number: 24775219.9
(22) Date of filing: 21.03.2024
(51) Int. Cl.: C12N 9/26, C12N 15/63, A61K 38/47

(54) **NOVEL HYALURONIDASE VARIANTS AND PHARMACEUTICAL COMPOSITION COMPRISING SAME**

(30) Priority: 23.03.2023 KR 20230038084
(71) Applicant: Alteogen, Inc., Daejeon 34054 (KR)
(72) Inventor: PARK, Soon Jae, Daejeon 34054 (KR); KIM, Kyuwan, Daejeon 34054 (KR); SONG, Hyung-Nam, Daejeon 34054 (KR)
(74) Representative: Mewburn Ellis LLP
(86) International application number: PCT/KR2024/003567
(87) International publication number: WO 2024/196173

(57) **Abstract**

The present invention relates to the technical field of protein engineering which increases the enzymatic activity at neutral pH of human hyaluronidase which is an enzyme that hydrolyzes hyaluronic acid, and relates to novel hyaluronidase variants or fragments thereof, which comprise one or more amino acid residue substitutions in an enzyme active region and the adjacent regions thereof among the amino acid sequence of native hyaluronidase Hyal1 of SEQ ID NO: 1 and in which there is selectively additional truncation of amino acid residues at the N-terminus and/or the C-terminus.

## Description

### [Technical Field]

The present invention relates to a novel human hyaluronidase variant, which is a hyaluronic acid hydrolyzing enzyme with improved activity at neutral pH, and more particularly, to a hyaluronidase variant or fragment thereof, which includes one or more amino acid residue substitutions at or adjacent to the active site of a native hyaluronidase having any one amino acid sequence selected from among SEQ ID NO: 1 to SEQ ID NO: 4, and additional amino acid residue truncation at the N-terminus or C-terminus, a method of producing the same, and a pharmaceutical composition including the same.

### [Background Art]

Human skin consists of the epidermis, dermis, and subcutaneous adipose layer, and there are six types of glycosaminoglycans in the skin. Glycosaminoglycans include hyaluronic acid, chondroitin sulfate, dermatan sulfate, heparan sulfate, heparin, and keratin sulfate.

Glycosaminoglycans are structures in which disaccharide-type sugars are repeatedly linked. The length of the sugars varies among glycosaminoglycans, but may range from hundreds to thousands of units. Among glycosaminoglycans, more than half of the hyaluronic acid present in the body is found in the skin. Hyaluronic acid is synthesized by hyaluronan synthase present in the cell membrane, exists alone without being bound to proteoglycan, and is the only glycosaminoglycan without a sulfate group. The other glycosaminoglycans are bound to proteoglycans and contain sulfate groups. Hyaluronic acid consists of glucuronic acid and N-acetylglucosamine linked by β-1,3 and β-1,4 bonds, and these disaccharides are repeated approximately 5,000 times. It is known that approximately 1/3 (5 g) of hyaluronic acid in the human body is broken down every day.

Hyaluronidase is an enzyme that breaks down hyaluronic acid located in the extracellular matrix. Six types of hyaluronidases are known to be present in humans. The six types of hyaluronidases are Hyall, Hyal2, Hyal3, Hyal4, HyalPS1, and PH20/SPAM1. In humans, Hyall and Hyal2 are hyaluronidases expressed in most tissues and are known to be present in plasma. Native PH20/SPAM1 (hereinafter referred to as PH20) is expressed in the cell membrane and acrosome membrane of sperm. However, HyalPS1, which is a pseudogene, is not expressed. Native human Hyal1 is expressed in lysosomes within cells and most tissues, including the liver, kidneys, heart, etc., and is composed of the amino acid sequence of SEQ ID NO: 1, and the mature form consists of amino acids F22 to W435 in the amino acid sequence of SEQ ID NO: 1 and is a protein with excellent enzyme activity at acidic pH. Native human Hyal2 is expressed in most tissues and consists of the amino acid sequence of SEQ ID NO: 2, and the mature form consists of amino acids M21 to G448 in the sequence of SEQ ID NO: 2 and has enzyme activity that hydrolyzes high-molecular-weight hyaluronic acid into a size of about 20 kDa. Native human Hyal3 is present in skin, bone marrow, testes, etc. and consists of the amino acid sequence of SEQ ID NO: 3, and the mature form consists of amino acids Q21 to V417 in the sequence of SEQ ID NO: 3 and has very weak enzyme activity under *in vitro* conditions. Native human Hyal4 is distributed in the placenta and muscles, consists of the amino acid sequence of SEQ ID NO: 4, and functions as a membrane protein. Native human PH20 is expressed in the testis and sperm and consists of the amino acid sequence of SEQ ID NO: 5, and the mature form consists of amino acids L36 to S490 in the sequence of SEQ ID NO: 5 and has excellent enzyme activity even at neutral pH.

Hyaluronidases are divided into three types depending on the way they cleave hyaluronic acid. These three types are enzymes that cleave the β-1,4 bond between N-acetylglucosamine and glucuronic acid using H₂O (EC 3.2.1.35), enzymes that cleave the β-1,3 bond (EC 3.2.1.36), and bacterial hyaluronidases that cleave the β-1,4 bond without using H₂O (EC 4.2.99.1).

The catalytic amino acids of Hyall are D129 and E131, and serve to hydrolyze hyaluronic acid by substrate-assisted catalysis. Hyal1 exhibits optimal activity at acidic pH 3-4, and has no enzyme activity at pH 4.5 or higher. Unlike Hyall, PH20 exhibits enzyme activity over a wide pH range of pH 3 to 8.

Arming et al. revealed that the catalytic amino acids of PH20 are D111 and E113 (Arming et al., 1997). Arming et al. designated the first amino acid, Leu, of the mature PH20 protein as position 1, so the catalytic amino acids in full-length PH20 correspond to D146 and E148.

Hyaluronidase hydrolyzes hyaluronic acid, thereby reducing the viscosity of hyaluronic acid in the extracellular matrix and increasing the permeability of the tissue (skin) to external substances. Since the subcutaneous area of the skin is neutral with a pH of about 7.0 to 7.5, PH20 is widely used in clinical practice among various types of hyaluronidases (Bookbinder et al., 2006). Examples of clinical use of PH20 include ocular relaxants and anesthetic injection additives in ophthalmic surgery, and PH20 is also co-administered when subcutaneously injecting antibody therapeutics (Bookbinder et al., 2006). In addition, by utilizing the characteristic of hyaluronidase being overexpressed in tumor cells, PH20 is used to hydrolyze hyaluronic acid in the extracellular matrix of tumor cells to increase the accessibility of anticancer drugs to tumor cells. Moreover, PH20 is used to promote reabsorption of excess fluid and blood within the tissues.

PH20 was first identified in guinea pig sperm by Lathrop et al., and is known to be expressed in sperm of many other species. The human PH20 gene was cloned by Lin et al. and Gmachl et al. The native human PH20 of SEQ ID NO: 5 is 60% identical in amino acid sequence to the guinea pig PH20 gene. The human PH20 enzyme is encoded by the SPAM1 (sperm adhesion molecule-1) gene and is present in a form in which Ser490 of PH20 is bound to GPI (glycosylphosphatidylinositol) on the surface of the sperm cell membrane and the inner side of the acrosome membrane. When sperm penetrate into the egg through the cumulus layer of the egg, which is rich in hyaluronic acid, hyaluronic acid is hydrolyzed using PH20. PH20 is present in less than 1% of the protein content in sperm and has six N-glycosylation sites (N82, N166, N235, N254, N368, and N393).

PH20 currently in commercial use is a form extracted from the testes of cattle or sheep, and includes Amphadase as bovine hyaluronidase and Vitrase as sheep hyaluronidase and etc.

Bovine testicular hyaluronidase (BTH) is a form of bovine native PH20 from which the signal peptide and the C-terminal 56 amino acids are removed by a protein post-translational modification process. BTH is also a glycoprotein, and of the total components including amino acids, mannose accounts for 5% and glucosamine accounts for 2.2%. Neutralizing antibodies may be produced by repeated administration of high doses of animal-derived hyaluronidase to humans. Furthermore, hyaluronidase derived from animals contains other biological materials in addition to PH20, which may cause allergic reaction when administered to humans (Bookbinder et al., 2006). In particular, production and use of PH20 extracted from cattle are restricted due to concerns about mad cow disease. To alleviate these problems, research has been conducted on recombinant proteins of human PH20.

Recombinant proteins of human PH20 have been reported to be expressed in yeast (*P. pastoris*), DS-2 insect cells, animal cells, and the like. Recombinant PH20 proteins produced in insect cells and yeast differ from human PH20 in the pattern of N-glycosylation during protein post-translational modification.

Among hyaluronidases, the protein structures of Hyall (PDB ID: 2PE4) (Chao et al., 2007) and Bee venom hyaluronidase (PDB ID: 1FCQ, 1FCU, 1FCV) have been elucidated. Hyall consists of two domains, a catalytic domain and an EGF-like domain, and the catalytic domain forms a (β/α)₈ configuration with eight repetitions of each alpha helix and beta strand, which characterize the secondary structure of the protein (Chao et al., 2007). The EGF-like domain is conserved in all C-terminally differently spliced variants of Hyall. The amino acid sequences of Hyal1 and PH20 are 35.1% identical, but the protein structure of PH20 has not yet been elucidated.

Recombinant protein of human PH20 was developed by Halozyme Therapeutics and is sold under the trade name Hylenex (Bookbinder et al., 2006; Frost, 2007).

When the catalytic amino acids D146 and E148 of PH20 were mutated to asparagine (D146N) and glutamine (E148Q), respectively, the enzyme activity was lost (Arming et al., 1997). Furthermore, substituting R246 of PH20 with glycine resulted in a 90% decrease in enzyme activity, and substituting E319 with glutamine and R322 with threonine resulted in loss of enzyme activity. A PH20 variant with C-terminal 36 amino acids deleted (474 to 509 amino acid truncation) showed a 75% decrease in enzyme activity compared to native PH20. This mutant was not secreted outside the cells but remained within HeLa cells. A PH20 variant with C-terminal 134 amino acids deleted had no enzyme activity of PH20 and was not secreted outside the cells. According to Frost et al., the C-terminal region 477 to 483 of PH20 is essential for soluble expression (Frost, 2007). The activity of full-length PH20 (1 to 509) or a PH20 variant with the C-terminus truncated at 467 (1 to 467) was only 10% of the activity of a PH20 variant with the C-terminus truncated at any one position of 477 to 483 (Frost, 2007).

Meanwhile, recombinant PH20 still lacks sufficient thermal stability or expression level, so there is a great demand for hyaluronidase with more improved characteristics, and there is a potential risk of safety-related problems occurring when a protein that exists only in the testis or sperm in the human body is used in the skin or other tissues. Hence, recombinant proteins using Hyall, which is expressed in most tissues in the human body, are needed, but these enzymes are only active at acidic pH, making availability thereof limited.

### [Citation List]

### [Non-Patent Literature]

Arming, S., Strobl, B., Wechselberger, C., and Kreil, G. (1997). In vitro mutagenesis of PH-20 hyaluronidase from human sperm. Eur J Biochem 247, 810-814.
Bookbinder, L.H., Hofer, A., Haller, M.F., Zepeda, M.L., Keller, G.A., Lim, J.E., Edgington, T.S., Shepard, H.M., Patton, J.S., and Frost, G.I. (2006). A recombinant human enzyme for enhanced interstitial transport of therapeutics. J Control Release 114, 230-241.
Chao, K.L., Muthukumar, L., and Herzberg, O. (2007). Structure of human hyaluronidase-1, a hyaluronan hydrolyzing enzyme involved in tumor growth and angiogenesis. Biochemistry 46, 6911-6920.
Frost, G.I. (2007). Recombinant human hyaluronidase (rHuPH20): an enabling platform for subcutaneous drug and fluid administration. Expert Opin Drug Deliv 4, 427-440.

### [Summary of Invention]

An object of the present invention is to provide a novel hyaluronidase variant or fragment thereof having a structure similar to a native hyaluronidase, preferably a mature native hyaluronidase, and being active at neutral pH.

Another object of the present invention is to provide a therapeutic composition including the novel hyaluronidase variant or fragment thereof and a therapeutic method using the same.

In order to accomplish the above objects, the present invention provides a novel hyaluronidase variant or fragment thereof having improved activity at neutral pH, which includes one or more amino acid residue substitutions at the enzyme active site and/or the substrate binding site or their adjacent regions in the amino acid sequence of a native hyaluronidase, preferably a mature native hyaluronidase, and additionally has truncation of a portion of the amino acid residues at the N-terminus and/or C-terminus.

The present invention also provides a composition for treating cancer including the novel hyaluronidase variant or fragment thereof according to the present invention and a treatment method using the same.

The present invention refers to a novel hyaluronidase variant or fragment thereof, including one or more amino acid residue substitutions in an enzyme active site, a substrate binding site, and/or their adjacent regions of a native hyaluronidase, and exhibiting hyaluronidase activity at neutral pH.

Also, the present invention refers to the novel hyaluronidase variant or fragment thereof, in which the native hyaluronidase may be a mature human native hyaluronidase.

Also, the present invention refers to the novel hyaluronidase variant or fragment thereof, in which the mature human native hyaluronidase may be a form of a human native hyaluronidase from which a signal peptide is removed.

Also, the present invention referes to the novel hyaluronidase variant or fragment thereof, in which the native hyaluronidase may be human native Hyall.

Preferably, the human native Hyall or fragment thereof has the amino acid sequence of SEQ ID NO: 1, or has the amino acid sequence of F22 to W435 in the sequence of SEQ ID NO: 1, but is not limited thereto.

Also, the present invention refers to the novel hyaluronidase variant or fragment thereof, in which the novel hyaluronidase variant may exhibit hyaluronidase activity at neutral pH.

The present invention refers to the novel hyaluronidase variant or fragment thereof, in which the enzyme active site may be D129 and/or E131 of SEQ ID NO: 1.

Also, the present invention refers to the novel hyaluronidase variant or fragment thereof, which may include amino acid substitution at one or more positions selected from the group consisting of Q41, W42, G63, S76, S77, Q78, T81, F93, R108, F110, Q111, A115, A116, A132, F139, D142, T143, R148, V170, Q172, G177, A181, Q188, R191, Y210, F212, L213, S214, N216, T218, Q220, Q228, P249, V251, Q263, A267, Q288, D292, T293, T294, H296, H305, L307, L356, R372, L379, G396, A403, M412, E415, and K417 in the amino acid sequence of SEQ ID NO: 1, or at one or more of amino acid positions corresponding to the amino acids in the amino acid sequence of mature human Hyall.

Preferably, the present invention refers to the novel hyaluronidase variant or fragment thereof, which includes amino acid substitution at one or more positions selected from the group consisting of S76, S77, Q78, A132, D142, T143, Y210, F212, L213, P249, V251, Q288, and D292 in the amino acid sequence of SEQ ID NO: 1, or at one or more amino acid positions corresponding to the amino acids in the amino acid sequence of mature human Hyall.

More preferably, the present invention refers to the novel hyaluronidase variant or fragment thereof, which includes amino acid residue substitution at D142 or P249 in the amino acid sequence of SEQ ID NO: 1, and optionally further includes amino acid substitution at one or more positions selected from the group consisting of S77, Q78, A132, D142, T143, Y210, F212, P249, and V251, or at one or more of the amino acid positions corresponding to the amino acids in the amino acid sequence of mature human Hyall.

Specifically, the present invention refers to the novel hyaluronidase variant or fragment thereof, which may include one or more amino acid substitutions selected from the group consisting of Q41E, W42F, G63R, S76V, S77D, Q78R, T81Y, F93N, R108K, F110K, Q111K, A115F, A116Y, A132E, F139R, D142K, T143P, R148K, V170K, Q172K, G177K, A181D, Q188K, R191K, Y210H, F212Y, L213K, S214K, N216G, T218N, Q220S, Q228R, P249N, V251Q, Q263R, A267R, Q288R, D292T, T293D, T294Q, H296K, H305Y, L307F, L356K, R372K, L379Y, G396K, A403K, M412F, E415K, and K417Y in the amino acid sequence of SEQ ID NO: 1, or amino acid substitution at one or more amino acid positions corresponding to the amino acids in the amino acid sequence of mature human Hyall.

Preferably, the present invention refers to the novel hyaluronidase variant or fragment thereof, which includes one or more amino acid substitutions selected from the group consisting of S76V, S77D, Q78R, A132E, D142K, T143P, Y210H, F212Y, L213K, P249N, V251Q, Q288R, and D292T in the amino acid sequence of SEQ ID NO: 1, or amino acid substitution at one or more amino acid positions corresponding to the amino acids in the amino acid sequence of mature human Hyall.

More preferably, the present invention refers to the novel hyaluronidase variant or fragment thereof, which includes amino acid residue substitution of D142K or P249N in the amino acid sequence of SEQ ID NO: 1, and optionally further includes one or more amino acid substitutions selected from the group consisting of S77D, Q78R, A132E, D142K, T143P, Y210H, F212Y, P249N, and V251Q, or amino acid substitution at one or more amino acid positions corresponding to the amino acids in the amino acid sequence of mature human Hyall.

In addition, the present invention refers to a composition for treating a disease, preferably cancer, including the novel hyaluronidase variant or fragment thereof described above.

In addition, the present invention refers to a nucleic acid encoding the novel hyaluronidase variant or fragment thereof described above.

In addition, the present invention refers to a recombinant expression vector including the nucleic acid.

In addition, the present invention relates to a host cell transformed with the recombinant expression vector.

In addition, the present invention refers to a host cell, which is selected from the group consisting of an animal cell, a plant cell, yeast, *Escherichia coli,* and an insect cell.

In addition, the present invention refers to a method of producing a novel hyaluronidase variant or fragment thereof, including culturing the host cell.

### [Brief Description of Drawings]

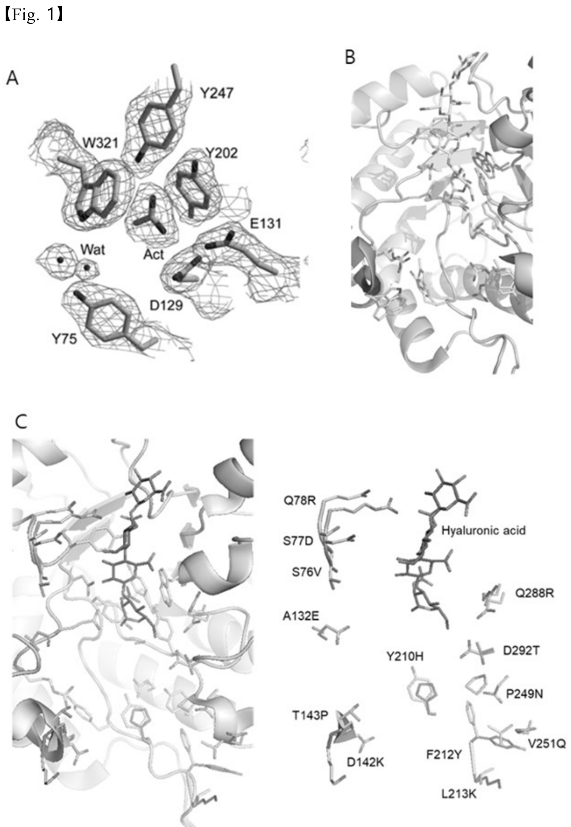

FIG. 1 shows results of analysis of protein structures of hyaluronidase Hyall and hyaluronidase PH20, which are superimposed. FIG. 1A shows results confirming that the active sites of Hyall and PH20 are the same, FIG. 1B shows results confirming that the binding sites of the substrate hyaluronic acid in Hyall and PH20 are the same, and FIG. 1C shows results confirming amino acids closely involved in enzyme activity and/or substrate binding in their adjacent regions of the active sites and/or substrate binding sites of Hyall and PH20;

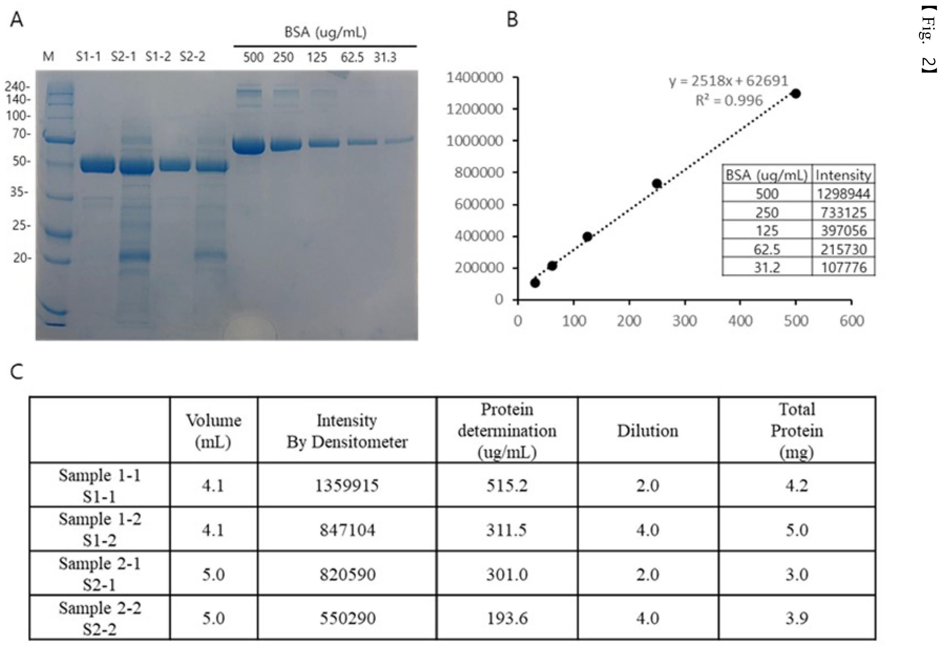

FIG. 2 shows results of quantitative analysis using SDS-PAGE of samples obtained by producing and purifying recombinant hyaluronidase Hyall and Hyal1-E131H variant, in which information about the samples is described in Example 1. FIG. 2A shows results of SDS-PAGE of purified samples, FIG. 2B shows a quantitative curve created using BSA, and FIG. 2C shows results of quantitative analysis of purified samples 1 and 2.

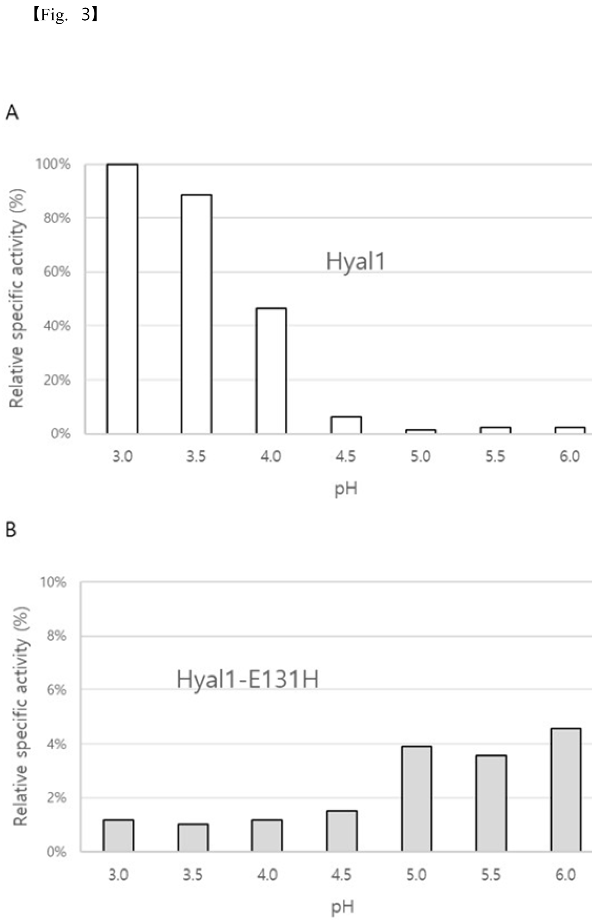

FIG. 3 shows results of measuring the enzyme activity of recombinant hyaluronidase Hyall and recombinant hyaluronidase variant Hyal1-E131H depending on pH,
   (A) shows results of measuring the enzyme activity of Hyall depending on pH.
   (B) shows results of measuring the enzyme activity of Hyal1-E131H depending on pH.

### [Detailed Descriptions and Specific Examples of Invention]

Unless otherwise defined, all technical and scientific terms used herein have the same meanings as typically understood by those skilled in the art to which the present invention belongs. In general, the nomenclature used herein is well known in the art and is typical.

The present invention provides a hyaluronidase variant or fragment thereof, which includes one or more amino acid residue substitutions at a site corresponding to an active site and/or a linking site thereof, preferably at and/or the adjacent regions of D129 and E131 of Hyall, in the amino acid sequence of a native hyaluronidase, preferably a mature native hyaluronidase, and additionally has truncation of a portion of amino acid residues at the N-terminus and/or C-terminus.

In the present invention, the position of the amino acid residue of each variant depends on the position of the amino acid of native hyaluronidase Hyall according to SEQ ID NO: 1.

Also, in the present invention, "mature native hyaluronidase" refers to a protein that consists of amino acid residues F22 to W435 of SEQ ID NO: 1 resulting from deletion of the signal peptide M1-G21 from the amino acid sequence of the native hyaluronidase Hyall of SEQ ID NO: 1.

In the present invention, based on the protein tertiary structure of human hyaluronidase Hyall (SEQ ID NO: 1), amino acids located at the active sites D129 and E131 and in the adjacent regions thereof were selected and mutations were attempted to induce catalysis of the enzyme even at neutral pH. In particular, the E131H variant shows a tendency of increased activity at pH 6 (FIG. 3). In the present invention, the possibility of providing a novel hyaluronidase variant or fragment thereof, which is different from native PH20 and variants thereof, was elucidated through various mutations in the active site of human hyaluronidase Hyall and/or the substrate binding site and/or the adjacent regions thereof.

Therefore, the novel hyaluronidase variant according to the present invention is characterized in that, in the native hyaluronidase Hyall (hyaluronidase having the amino acid sequence of SEQ ID NO: 1), preferably mature native hyaluronidase Hyall (a protein that consists of F22 to W435 in the amino acid sequence of SEQ ID NO: 1), the active site D129 and/or E131 and/or some amino acid residues adjacent thereto are substituted with other amino acid residues, but the present invention is not limited thereto.

The X-ray diffraction crystal structure of hyaluronidase Hyall has been determined and the protein structure is deposited in the public database Protein data bank (https://www.rcsb.org/) under the ID number: 2PE4. In addition, hyaluronidase PH20, which is active at neutral pH, does not have an X-ray diffraction crystal structure, but the protein structure estimated by DeepMind (USA) and the European Bioinformatics Institute (EMBL-EBI) is deposited in the public database AlphaFold protein structure database (https://alphafold.ebi.ac.uk/). A structural comparison study on the active sites of these proteins was conducted as shown in FIG. 1, through which possible amino acid substitution variants were obtained, and follow-up studies thereon were conducted to achieve the purpose of the present invention.

The amino acid sequences of native human Hyall, Hyal2, Hyal3, Hyal4, and PH20 are shown in Table 1 below.

**[Table 1]**

| SEQ ID NO: | Descriptio n | Sequence |
|---|---|---|
| 1 | Hyal1 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ |
| | | LTPGGGPLSL RGALSLEDQA QMAVEFKCRC YPGWQAPWCE RKSMW |
| 2 | Hyal2 | MRAGPGPTVT LALVLAVSWA MELKPTAPPI FTGRPFVVAW DVPTQDCGPR LKVPLDLNAF DVQASPNEGF VNQNITIFYR DRLGLYPRFD SAGRSVHGGV PQNVSLWAHR KMLQKRVEHY IRTQESAGLA VIDWEDWRPV WVRNWQDKDV YRRLSRQLVA SRHPDWPPDR IVKQAQYEFE FAAQQFMLET LRYVKAVRPR HLWGFYLFPD CYNHDYVQNW ESYTGRCPDV EVARNDQLAW LWAESTALFP SVYLDETLAS SRHGRNFVSF RVQEALRVAR THHANHALPV YVFTRPTYSR RLTGLSEMDL ISTIGESAAL GAAGVILWGD AGYTTSTETC QYLKDYLTRL LVPYVVNVSW ATQYCSRAQC HGHGRCVRRN PSASTFLHLS TNSFRLVPGH APGEPQLRPV GELSWADIDH LQTHFRCQCY LGWSGEQCQW DHRQAAGGAS EAWAGSHLTS LLALAALAFT WTL |
| 3 | Hyal3 | MTTQLGPALV LGVALCLGCG QPLPQVPERP FSVLWNVPSA HCEARFGVHL PLNALGIIAN RGQHFHGQNM TIFYKNQLGL YPYFGPRGTA HNGGIPQALP LDRHLALAAY QIHHSLRPGF AGPAVLDWEE WCPLWAGNWG RRRAYQAASW AWAQQVFPDL DPQEQLYKAY TGFEQAARAL MEDTLRVAQA LRPHGLWGFY HYPACGNGWH SMASNYTGRC HAATLARNTQ LHWLWAASSA LFPSIYLPPR LPPAHHQAFV RHRLEEAFRV ALVGHRHPLP VLAYVRLTHR RSGRFLSQDD LVQSIGVSAA LGAAGVVLWG DLSLSSSEEE CWHLHDYLVD TLGPYVINVT RAAMACSHQR CHGHGRCARR DPGQMEAFLH LWPDGSLGDW KSFSCHCYWG WAGPTCQEPR PGPKEAV |
| 4 | Hyal4 | MKVLSEGQLK LCVVQPVHLT SWLLIFFILK SISCLKPARL PIYQRKPFIA AWNAPTDQCL IKYNLRLNLK MFPVIGSPLA KARGQNVTIF YVNRLGYYPW YTSQGVPING GLPQNISLQV HLEKADQDIN YYIPAEDFSG LAVIDWEYWR PQWARNWNSK DVYRQKSRKL ISDMGKNVSA TDIEYLAKVT FEESAKAFMK ETIKLGIKSR PKGLWGYYLY PDCHNYNVYA PNYSGSCPED EVLRNNELSW LWNSSAALYP SIGVWKSLGD SENILRFSKF RVHESMRIST MTSHDYALPV FVYTRLGYRD EPLFFLSKQD LVSTIGESAA LGAAGIVIWG DMNLTASKAN CTKVKQFVSS DLGSYIANVT RAAEVCSLHL CRNNGRCIRK MWNAPSYLHL NPASYHIEAS EDGEFTVKGK ASDTDLAVMA DTFSCHCYQG YEGADCREIK TADGCSGVSP SPGSLMTLCL LLLASYRSIQL |
| 5 | PH20 | MGVLKFKHIF FRSFVKSSGV SQIVFTFLLI PCCLTLNFRA PPVIPNVPFL WAWNAPSEFC LGKFDEPLDM SLFSFIGSPR INATGQGVTI FYVDRLGYYP YIDSITGVTV NGGIPQKISL QDHLDKAKKD ITFYMPVDNL GMAVIDWEEW RPTWARNWKP KDVYKNRSIE LVQQQNVQLS LTEATEKAKQ EFEKAGKDFL VETIKLGKLL RPNHLWGYYL FPDCYNHHYK KPGYNGSCFN |
| | | VEIKRNDDLS WLWNESTALY PSIYLNTQQS PVAATLYVRN RVREAIRVSK IPDAKSPLPV FAYTRIVFTD QVLKFLSQDE LVYTFGETVA LGASGIVIWG TLSIMRSMKS CLLLDNYMET ILNPYIINVT LAAKMCSQVL CQEQGVCIRK NWNSSDYLHL NPDNFAIQLE KGGKFTVRGK PTLEDLEQFS EKFYCSCYST LSCKEKADVK DTDAVDVCIA DGVCIDAFLK PPMETEEPQI FYNASPSTLS ATMFIVSILF LIISSVASL |

In the present invention, a representation in which a one-letter amino acid residue name and a number are written together, such as "E131", means an amino acid residue at each position in the amino acid sequence according to the corresponding sequence number. For example, "E131" in SEQ ID NO: 1 means that the amino acid residue at position 131 in the amino acid sequence is glutamic acid.

Also, "E131H" in SEQ ID NO: 1 means that glutamic acid, which is the amino acid at position 131 in SEQ ID NO: 1, is substituted with histidine.

The amino acids involved in the active site of hyaluronidase Hyall are known to be Y75, Y202, Y247, and W321, in addition to D129 and E131 (Chao K.L. 2007), and these are also conserved in another hyaluronidase, PH20. Also, amino acids involved in binding the substrate hyaluronic acid are conserved in both proteins. The active site and the substrate binding site and the amino acids adjacent to these two sites may influence the pH environment for enzyme activity.

The novel hyaluronidase variant according to the present invention may include variation at any position in the amino acid sequence of hyaluronidase Hyall of SEQ ID NO: 1, but preferably mutation, particularly amino acid substitution, is possible at one or more positions selected from the group consisting of Q41, W42, G63, S76, S77, Q78, T81, F93, R108, F110, Q111, A115, A116, A132, F139, D142, T143, R148, V170, Q172, G177, A181, Q188, R191, Y210, F212, L213, S214, N216, T218, Q220, Q228, P249, V251, Q263, A267, Q288, D292, T293, T294, H296, H305, L307, L356, R372, L379, G396, A403, M412, E415, and K417, preferably at one or more positions selected from the group consisting of S76, S77, Q78, A132, D142, T143, Y210, F212, L213, P249, V251, Q288, and D292, but is not limited thereto.

More preferably, the present invention relates to a novel hyaluronidase variant or fragment thereof, in which the one or more amino acid residue substitutions include amino acid residue substitution at D142 or P249 in the amino acid sequence of SEQ ID NO: 1, and optionally further includes amino acid substitution at one or more positions selected from the group consisting of S77, Q78, A132, D142, T143, Y210, F212, P249, and V251.

More preferably, the novel hyaluronidase variant according to the present invention includes one or more amino acid substitutions selected from the group consisting of Q41E, W42F, G63R, S76V, S77D, Q78R, T81Y, F93N, R108K, F110K, Q111K, A115F, A116Y, A132E, F139R, D142K, T143P, R148K, V170K, Q172K, G177K, A181D, Q188K, R191K, Y210H, F212Y, L213K, S214K, N216G, T218N, Q220S, Q228R, P249N, V251Q, Q263R, A267R, Q288R, D292T, T293D, T294Q, H296K, H305Y, L307F, L356K, R372K, L379Y, G396K, A403K, M412F, E415K, and K417Y in the amino acid sequence of native hyaluronidase Hyall of SEQ ID NO: 1, more preferably one or more amino acid substitutions selected from the group consisting of S76V, S77D, Q78R, A132E, D142K, T143P, Y210H, F212Y, L213K, P249N, V251Q, Q288R, and D292T, but is not limited thereto.

Much more preferably, the novel hyaluronidase variant according to the present invention includes amino acid residue substitution of D142K or P249N in the amino acid sequence of SEQ ID NO: 1, and optionally further includes one or more amino acid substitutions selected from the group consisting of S77D, Q78R, A132E, D142K, T143P, Y210H, F212Y, P249N, and V251Q.

The hyaluronidase variant according to the present invention is also understood to include variants in which amino acid residues are conservatively substituted at the specific amino acid residue positions.

As used herein, "conservative substitution" refers to modification of a variant that includes substituting one or more amino acids with other amino acids having similar biochemical properties that do not result in loss of biological or biochemical function of the variant.

"Conservative amino acid substitution" is substitution that replaces an amino acid residue with other amino acid residues having a similar side chain. Classes of amino acid residues with similar side chains have been defined in the art and are well known. These classes include amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine), amino acids with non-polar side chains (e.g., alanine, valine, leucine, isoleucine, proline, phenylalanine, methionine, tryptophan), amino acids with beta-branched side chains (e.g., threonine, valine, isoleucine), and amino acids with aromatic side chains (e.g., tyrosine, phenylalanine, tryptophan, histidine).

It is expected that the hyaluronidase variants of the present invention may still retain activity even when having conservative amino acid substitutions.

The novel hyaluronidase variant according to the present invention may be, but is not limited to, a fragment in which some amino acids are deleted, for example, a fragment in which a signal peptide is deleted or removed.

Preferably, the human native Hyall or fragment thereof has the amino acid sequence of SEQ ID NO: 1, or the amino acid sequence of F22 to W435 in the sequence of SEQ ID NO: 1, and additionally includes the amino acid substitution described above, but is not limited thereto.

Another aspect of the present invention provides a composition for treating a disease including the hyaluronidase variant according to the present invention and a method of treating a disease using the same.

The composition for treating the disease may be a pharmaceutical composition. The pharmaceutical composition may further include a pharmaceutically acceptable carrier, and the carrier may be one or more selected from the group consisting of, but is not limited to, those commonly employed in the formulation of drugs, such as lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like. The pharmaceutical composition may further include one or more selected from the group consisting of diluents, excipients, lubricants, wetting agents, sweeteners, flavoring agents, emulsifiers, suspending agents, and preservatives commonly employed in the manufacture of pharmaceutical compositions.

The pharmaceutical composition may be administered orally or parenterally. Parenteral administration may include intravenous injection, subcutaneous injection, intramuscular injection, intraperitoneal injection, endothelial administration, topical administration, intranasal administration, intrapulmonary administration, and intrarectal administration. Since proteins or peptides are digested when administered orally, oral compositions may be formulated to coat the active agent or protect the same from degradation in the stomach. Furthermore, the composition may be administered by any device capable of transporting the active agent to a target cell.

The pharmaceutical composition may be in the form of a solution, suspension, syrup, or emulsion in oil or aqueous medium, or may be formulated in the form of extracts, fine powders, powders, granules, tablets, or capsules, and may further include a dispersant or a stabilizer for formulation.

In particular, the therapeutic composition according to the present invention may be used alone or in combination with other therapeutic agents.

Still another aspect of the present invention relates to a nucleic acid encoding the hyaluronidase variant or variants thereof according to the present invention.

The nucleic acid used herein may be present in cells or cell lysates, or may be present in a partially purified or substantially pure form. A nucleic acid is "isolated" or "rendered substantially pure" when purified from other cellular components or other contaminants, such as nucleic acids or proteins of other cells, by standard techniques, including alkaline/SDS treatment, CsCl banding, column chromatography, agarose gel electrophoresis, and others well known in the art. The nucleic acid of the present invention may be, for example, DNA or RNA.

Yet another aspect of the present invention relates to a vector including the nucleic acid. For expression of the hyaluronidase variant or fragment thereof according to the present invention, DNA encoding the hyaluronidase variant may be obtained by standard molecular biology techniques (e.g., PCR amplification or cDNA cloning using a hybridoma expressing the hyaluronidase variant), and DNA may be "operably linked" to transcriptional and translational control sequences and thus inserted into an expression vector.

As used herein, the term "operably linked" may mean that a gene encoding the hyaluronidase variant or fragment thereof is ligated into a vector such that the transcriptional and translational control sequences within the vector perform their intended function of regulating transcription and translation of the gene encoding the hyaluronidase variant or fragment thereof. Expression vectors and expression control sequences are selected to be compatible with the expression host cell used. A gene encoding the hyaluronidase variant is inserted into an expression vector by standard methods (e.g., ligation of a gene fragment encoding the hyaluronidase variant or fragment thereof and complementary restriction enzyme sites on the vector, or blunt-end ligation if no restriction enzyme sites are present at all).

Also, the recombinant expression vector carries a regulatory sequence that controls the expression of a gene encoding the hyaluronidase variant in a host cell. A "regulatory sequence" may include a promoter, enhancer, and other expression control elements (e.g., a polyadenylation signal) that control transcription or translation of a gene encoding the hyaluronidase variant or fragment thereof. Those skilled in the art will recognize that the design of the expression vector may vary by differently selecting a regulatory sequence depending on factors such as the choice of host cell to be transformed, the level of protein expression, etc.

Still yet another aspect of the present invention relates to a host cell including the nucleic acid or the vector. The host cell according to the present invention is preferably selected from the group consisting of, but is not limited to, an animal cell, a plant cell, yeast, *Escherichia coli,* and an insect cell.

Specifically, the host cell according to the present invention may be a prokaryotic cell such as *Escherichia coli, Bacillus subtilis,* Streptomyces sp., Pseudomonas sp., *Proteus mirabilis,* or Staphylococcus sp. The host cell may also be a eukaryotic cell, such as a cell of a higher eukaryote, for example, a fungus such as Aspergillus sp., yeast such as *Pichia pastoris, Saccharomyces cerevisiae,* Schizosaccharomyces sp., and *Neurospora crassa,* other lower eukaryotic cells, and a cell from an insect.

The host cell may also be derived from plants or mammals. Preferable examples thereof include, but are not limited to, monkey kidney cells 7 (COS7), NSO cells, SP2/0, Chinese hamster ovary (CHO) cells, W138, baby hamster kidney (BHK) cells, MDCK, myeloma cell lines, HuT 78 cells, and HEK293 cells, and particularly preferably, CHO cells are used.

The nucleic acid or the vector is transfected into a host cell. Any of a variety of techniques commonly used to introduce exogenous nucleic acids (DNA or RNA) into prokaryotic or eukaryotic host cells for "transfection" may be used, such as electrophoresis, calcium phosphate precipitation, DEAE-dextran transfection, or lipofection. A variety of expression host/vector combinations may be used to express the PH20 variant or fragment thereof according to the present invention. Examples of the expression vector suitable for a eukaryotic host include, but are not limited to, expression control sequences derived from SV40, bovine papillomavirus, adenovirus, adeno-associated virus, cytomegalovirus, and retrovirus. Examples of the expression vector useful for a bacterial host include bacterial plasmids obtained from *Escherichia coli,* such as pET, pRSET, pBluescript, pGEX2T, pUC vectors, col E1, pCR1, pBR322, pMB9 and derivatives thereof, plasmids having a broader host range such as RP4, phage DNA such as the very diverse phage lambda derivatives λ and ANM989, and other DNA phages such as M13 and filamentous single-stranded DNA phages. Examples of the expression vector useful for a yeast cell include 2°C plasmid and derivatives thereof. The vector useful for an insect cell is pVL941.

Even yet another aspect of the present invention relates to a method of producing the hyaluronidase variant or fragment thereof according to the present invention, including expressing the hyaluronidase variant or fragment thereof according to the present invention by culturing a host cell.

When a recombinant expression vector capable of expressing the hyaluronidase variant or fragment thereof is introduced into a mammalian host cell, the hyaluronidase variant or fragment thereof may be produced by culturing the host cell for a period of time sufficient to allow expression in the host cell, or more preferably for a period of time sufficient to allow the hyaluronidase variant to be secreted into a culture medium in which the host cell is cultured.

In some cases, the expressed hyaluronidase variant may be isolated from the host cell and purified to homogeneity. The separation or purification of the hyaluronidase variant may be performed by a separation or purification method commonly used for proteins, for example, chromatography. The chromatography may be, but is not limited to, a combination of one or more selected from among, for example, affinity chromatography, ion exchange chromatography, and hydrophobic chromatography. In addition to the chromatography, filtration, ultrafiltration, salting out, dialysis, etc. may be used in combination.

A better understanding of the present invention may be obtained through the following examples. These examples are merely set forth to illustrate the present invention and are not to be construed as limiting the scope of the present invention, as will be apparent to those skilled in the art.

### [Example 1] Production of hyaluronidase Hyall and variant thereof

### Example 1-1 Cloning of hyaluronidase Hyall and variant thereof

To produce recombinant Hyall, cDNA of native Hyall (clone ID: hMU005315) was purchased from the Korea Human Gene Bank. Mature native Hyall encodes amino acids F22 to W435. The Hyall gene was amplified using polymerase chain reaction (hereinafter referred to as PCR) and inserted into the XhoI and NotI restriction enzyme sites of the pcDNA3.4-TOPO vector. The signal peptide of human Hyall was used for expression in ExpiCHO cells. Additionally, a 6xHis-tag DNA sequence was placed at the 3'-end of Hyall cDNA for protein purification using a HisTrap column. Amino acid substitution in the Hyall variant was performed using a PCR method, and amino acid substitution was confirmed using DNA sequencing.

The primers used for Hyall cloning are listed in Table 2 below, and the specific sequences thereof are shown in Table 3 below.

**[Table 2]**

| Clone | Template | Primer | | |
|---|---|---|---|---|
| | | 1 | 2 | 3 |
| p4-B4001 | cDNA (hMU005315) | HYAL1-Xho | HYAL1-Not | - |
| p4-B4201 | cDNA (hMU005315) | HYAL1-Xho | HYAL1-6H-Not | |
| p4-B4201-131H | cDNA (hMU005315) | HYAL1-Xho | HYAL1-6H-Not | HYAL1-E131H |
| p4-BG4201-h2 | cDNA (hMU005315) | HYAL1-Xho | HYAL1-S76V-R | HYAL1-6H-Not |
| p4-BG4201-h3 | cDNA (hMU005315) | HYAL1-Xho | HYAL1-S77D-R | HYAL1-6H-Not |
| p4-BG4201-h4 | cDNA (hMU005315) | HYAL1-Xho | HYAL1-Q78R-R | HYAL1-6H-Not |
| p4-BG4201-h5 | cDNA (hMU005315) | HYAL1-Xho | HYAL1-A132E-R | HYAL1-6H-Not |
| p4-BG4201-h6 | cDNA (hMU005315) | HYAL1-Xho | HYAL1-D142K-R | HYAL1-6H-Not |
| p4-BG4201-h7 | cDNA (hMU005315) | HYAL1-Xho | HYAL1-T143P-R | HYAL1-6H-Not |
| p4-BG4201-h8 | cDNA (hMU005315) | HYAL1-Xho | HYAL1-Y210H-R | HYAL1-6H-Not |
| p4-BG4201-h9 | cDNA (hMU005315) | HYAL1-Xho | HYAL1-F212Y-R | HYAL1-6H-Not |
| p4-BG4201-h10 | cDNA (hMU005315) | HYAL1-Xho | HYAL1-L213-R | HYAL1-6H-Not |
| p4-BG4201-h11 | cDNA (hMU005315) | HYAL1-Xho | HYAL1-V251Q-F | HYAL1-6H-Not |
| p4-BG4201-h12 | cDNA (hMU005315) | HYAL1-Xho | HYAL1-P249N-F | HYAL1-6H-Not |
| p4-BG4201-h13 | cDNA (hMU005315) | HYAL1-Xho | HYAL1-Q288R-F | HYAL1-6H-Not |
| p4-BG4201-h14 | cDNA (hMU005315) | HYAL1-Xho | HYAL1-D292T-F | HYAL1-6H-Not |
| p4-BG4211 | Optimized DNA | opH1-Xho | opH1-6H-Not | |
| p4-BG4211-h3 | Optimized DNA | opH1-Xho | opH1-S77D-R | opH1-6H-Not |
| p4-BG4211-h6 | Optimized DNA | opH1-Xho | opH1-D142K-R | opH1-6H-Not |
| p4-BG4211-h8 | Optimized DNA | opH1-Xho | opH1-Y210H-R | opH1-6H-Not |
| p4-BG4211-h12 | Optimized DNA | opH1-Xho | opH1-P249N-F | opH1-6H-Not |
| p4-BG4211-h22 | Optimized DNA | opH1-Xho | opH1-D142K-R | opH1-6H-Not |
| p4-BG4211-h23 | Optimized DNA | opH1-Xho | opH1-S77D,Q78R-R | opH1-6H-Not |
| p4-BG4211-h24 | Optimized DNA | opH1-Xho | opH1-A132E-R | opH1-6H-Not |
| p4-BG4211-h25 | Optimized DNA | opH1-Xho | opH1-D142K,T143P-R | opH1-6H-Not |
| p4-BG4211-h26 | Optimized DNA | opH1-Xho | opH1-Y210H,F212Y-R | opH1-6H-Not |
| p4-BG4211-h27 | Optimized DNA | opH1-Xho | opH1-V251Q-F | opH1-6H-Not |
| p4-BG4211-h37 | Optimized DNA | opH1-Xho | opH1-Y210H-R | opH1-6H-Not |
| p4-BG4211-h38 | Optimized DNA | opH1-Xho | opH1-D142K-R | opH1-6H-Not |
| p4-BG4211-h39 | Optimized DNA | opH1-Xho | opH1-A132E-R | opH1-6H-Not |
| p4-BG4211-h40 | Optimized DNA | opH1-Xho | opH1-T86D-R | opH1-6H-Not |

**[Table 3]**

| Primer | SEQ ID NO: | Nucleic acid sequence (5'-> 3') |
|---|---|---|
| HYAL1-Xho | 6 | GAATATCTCGAGGCCACCATGGCAGCCCACCTGCTTCCCATCTG |
| HYAL1-Not | 7 | CTAATTGCGGCCGCTTATCACCACATGCTCTTCCGCTCAC |
| HYAL1-6H-Not | 8 | CTAATTGCGGCCGCTCATTAGTGGTGATGGTGATGATGCCACATGCTCTTC GCTC |
| HYAL1-E131H | 9 | TGAAGGCCCAGCGTGGGCGCCATGCATGCCAGTCGATGACTGCCAGCCCT |
| HYAL1-GSGS-h11-R | 10 | TGGGCACGGATGCCTGATGGGCACGAGCCGCTACCGCAGTCAGGGAAGCCA TAG |
| HYAL1-S76V-R | 11 | TAGGGGTAGGTGCCCAGCTGGGAGACATAGAAAATTGTCATGTCAG |
| HYAL1-S77D-R | 12 | TAGGGGTAGGTGCCCAGCTGGTCGCTATAGAAAATTGTCATGTC |
| HYAL1-Q78R-R | 13 | TAGTAGGGGTAGGTGCCCAGCCGGGAGCTATAGAAAATTGTCATG |
| HYAL1-A132E-R | 14 | TGAAGGCCCAGCGTGGGCGCCATTCCTCCCAGTCGATGACTGCCAG |
| HYAL1-D142K-R | 15 | TGCCGGTAAATGTCCTTGGTCTTCCAGTTGAAGGCCCAGCGTG |
| HYAL1-T143P-R | 16 | TGAGCGCTGCCGGTAAATGTCCTTAGGGTCCCAGTTGAAGGCCCAGC |
| HYAL1-Y210H-R | 17 | TAGTTGGGGCTTAGAAAGTCATGGTTGTAGCAGTCAGGGAAGC |
| HYAL1-F212Y-R | 18 | TGGCCGGTGTAGTTGGGGCTTAGATAGTCATAGTTGTAGCAGTCAG |
| HYAL1-L213-R | 19 | TGGCCGGTGTAGTTGGGGCTTTTAAAGTCATAGTTGTAGCAGTC |
| HYAL1-V251Q-F | 20 | ATCCCAGCATCTACATGCCCGCACAGCTGGAGGGCACAGGGAAGTC |
| HYAL1-P249N-F | 21 | TCTATCCCAGCATCTACATGAACGCAGTGCTGGAGGGCACAGGG |
| HYAL1-Q288R-F | 22 | ATCTGCCGGTGCTGCCCTATGTCCGGATCTTCTATGACACGACAAAC |
| HYAL1-D292T-F | 23 | TGCCCTATGTCCAGATCTTCTATACCACGACAAACCACTTTCTGCCCC |
| HYAL1-VAN-h15-R | 24 | TGGGAGCTATAGAAAATTGTTATGAACTGGCCGGTGGCGCTCTTCTGTGGG CTGCCTTTCACATCGAAGACACTGACATC |
| HYAL1-IDE-h16-R | 25 | TTCTGGGGCAGACCACCAAACACAGTGTTCCCAGTCTTTTCATCGATGTAG GGGTAGGTGCCCAGCTGGG |
| HYAL1-VFT-h17-F | 26 | ATCTGCCGGTGCTGCCCTATGTCCAGATCGTATTCACTGACGGGTCAAGCA CCTTTCTGCCCCTGGATGAGCTG |
| opH1-Xho | 27 | GAATATCTCGAGGCCACCATGGCAGCACATCTACTACC |
| opH1-6H-Not | 28 | CTCTAATTGCGGCCGCCTATCAGTGGTGATGGTGATGATGCCACATGGACT TCCGCTCGC |
| opH1-S77D-R | 29 | TAAGGGTAGGTTCCCAGTTGATCGGAGTAGAAGATGGTCATATC |
| opH1-D142K-R | 30 | TGTCTGTAGATGTCCTTGGTCTTCCAGTTGAAGGCCCATCTAG |
| opH1-Y210H-R | 31 | TATAGTTAGGGCTCAGAAAGTCGTGGTTGTAGCAGTCAGGGAAGCC |
| opH1-S77D,Q78 R-R | 32 | TAGTAAGGGTAGGTTCCCAGTCGATCGGAGTAGAAGATGGTCATATC |
| opH1-A132E-R | 33 | TGAAGGCCCATCTAGGCCGCCACTCCTCCCAGTCGATCACGGCCAG |
| opH1-D142K,T1 43P-R | 34 | TGTCTGTAGATGTCCTTGGGCTTCCAGTTGAAGGCCCATC |
| opH1-Y210H,F2 12Y-R | 35 | TGGCCGGTATAGTTAGGGCTCAGATAGTCGTGGTTGTAGCAGTCAG |
| opH1-V251Q-F | 36 | ACCCCTCCATCTACATGCCTGCCCAGCTGGAAGGCACCGGCAAGAGC |
| A1AT-B7H4-Xho | 37 | GAATATCTCGAGGCCACCATGCCTAGTTCCGTG |
| AT-HYAL1-R | 38 | TAGGCAGCAGGGGGCCTCTAAAAGCCAGTGAGACGGGGACCAG |
| ALB-SP-Xho | 39 | GAATATCTCGAGGCCACCATGAAGTGGGTTACA |
| hSA-HYAL1-R | 40 | TAGGCAGCAGGGGGCCTCTAAAAGAGTAAGCAGAGGAGAAAAG |
| Xho-P1 | 41 | CAATACCTCGAGGCCACCATGGCTACAGGCTCCCG |
| GH-HYAL1-R | 42 | TAGGCAGCAGGGGGCCTCTAAAGGCACTGCCCTCTTGAAGCC |
| 8H2-VL-Xho I -F | 43 | ACTAAGCTCGAGGCCACCATGGTGAGTAGTGCACAATTTC |
| Kappa-HYAL1-R | 44 | TAGGCAGCAGGGGGCCTCTAAAACATCTGGTGCCCTGGAAGC |
| opH1-P249N-F | 45 | AGCCCTGTACCCCTCCATCTACATGAATGCCGTGCTGGAAGGCACCGGC |
| opH1-NTQ-h36-F | 46 | AGCCCTGTACCCCTCCATCTACATGAATACCCAGCAGTCACCCGTCGCCGC GACCCTGTACGTGCAGCACAGAGTGGCC |
| opH1-T86D-R | 47 | TCCGCCGAACACAGGCTCGCCGGTAGGGTCGTAGTAAGGGTAGGTTCCCAG |

### Example 1-2 Purification of hyaluronidase Hyall and Hyal1-E131H variant

Hyall recombinant protein and E131H variant produced in ExpiCHO cells were purified by the following three-step column chromatography using an AKTA Prime system (Cytiva, USA).

The culture supernatant was adjusted to pH 10.0 by adding Tris, and then injected into Blue Sepharose 6 Fast Flow resin equilibrated with buffer A (10 mM glycine, pH 10). After washing away impurities using buffer A, the target protein was eluted using buffer B (10 mM glycine, 1 M NaCl, pH 10). For CM-650M column purification, the collected fractions were dialyzed against buffer C (50 mM NaPi, 0.1% Triton X-100, pH 6.0).

The fractions collected from the Blue Sepharose 6 Fast Flow column were injected into CM-650M resin equilibrated with buffer D (50 mM NaPi, 0.1% Triton X-100, pH 6.0). After washing away impurities using buffer D, the target protein was eluted using buffer E (50 mM NaPi, 0.1% Triton X-100, 0.5 M NaCl, pH 6.0). For DEAE Sepharose Fast Flow column purification, the collected fractions were dialyzed against buffer F (10 mM NaPi, pH 7.0).

Each fraction collected from the CM-650M column was injected into DEAE Sepharose Fast Flow resin equilibrated with buffer G (5 mM K₂HPO₄, pH 7.1). This process was performed in flow-through (FT) mode, and after completion of sample loading, buffer G was additionally allowed to flow to ensure that the target protein in the column was completely recovered. Thereafter, impurities were stripped using buffer H (5 mM K₂HPO₄, 1 M NaCl, pH 7.1). DEAE Sepharose Fast Flow column purification was performed twice for each fraction (#4-6, #8-14) collected in the CM-650M process, and the samples obtained from the FT section of each process were named Sample 1 and Sample 2. Each sample collected in the purification step was electrophoresed at 150 V for 1 hour using a 10% acrylamide gel.

Each of Sample 1 and Sample 2 collected from the DEAE Sepharose Fast Flow column was dialyzed against buffer I (20 mM NaPi, 77 mM NaCl, pH 7.0), and concentrated using a stirred cell. The concentrated sample was electrophoresed on a 10% acrylamide gel with bovine serum albumin (BSA) as a standard, and the concentration of the target protein was calculated using the band intensity calculated from the CS analyzer program [FIG. 2].

### Example 1-3 Purification of HIS-tagged hyaluronidase Hyall and variant thereof

The C-terminal His-tagged Hyall variant polypeptides produced in ExpiCHO cells were purified in two steps using an AKTA Prime system or a similar system (GE Healthcare), with Q Sepharose for anion exchange chromatography and HisTrap HP column for His-Tag affinity chromatography, which are column chromatography processes.

Buffer A (20 mM sodium phosphate, pH 7.5) and buffer B (20 mM sodium phosphate, pH 7.5, 0.5 M NaCl) were prepared for protein purification using a Q Sepharose column. The protein was bound to a Q Sepharose column, 5 CV of buffer A was allowed to flow to remove nonspecifically bound proteins, and 5 CV of buffer B was then allowed to flow with a concentration gradient of 0 to 100% to elute the protein.

Buffer A (20 mM sodium phosphate, 500 mM NaCl, pH 7.5) and buffer B (20 mM sodium phosphate, 500 mM NaCl, 500 mM imidazole, pH 7.5) were prepared for protein purification using a HisTrap HP column. After binding the protein sample to the HisTrap HP column, 7 CV of 7% buffer B was allowed to flow to remove nonspecifically bound proteins, and 3 CV of 40% buffer B was allowed to flow to elute the target protein. The column eluate was dialyzed using dialysis buffer (20 mM sodium phosphate, 100 mM NaCl, pH 7.0).

### [Example 2] Measurement of activity of hyaluronidase variant

Hyaluronidase activity was measured by a turbidity analysis method of measuring the extent of turbidity caused by the precipitate formed when hyaluronic acid is mixed with albumin (BSA) as absorbance. When hyaluronic acid is hydrolyzed by PH20 polypeptide, the turbidity/absorbance of the precipitate formed by mixing with albumin decreases. This analysis was typically performed at pH 5.3 as follows. Hyaluronidase standards with known activity (units) were diluted to 6, 8, 10, 12, 15, and 20 units/ml and prepared in individual test tubes. Purified protein samples were diluted in buffer (20 mM sodium phosphate, pH 5.3, 77 mM sodium chloride, and 0.01% (w/v) bovine serum albumin) by adjusting the dilution factor to fall within the range of the standard curve. 50 µl of the diluted sample was dispensed into each well of a 96-well plate followed by warming reaction at 37°C for 10 minutes. 50 µl of 0.06% hyaluronic acid was additionally dispensed into each well. 0.06% hyaluronic acid is dissolved in 300 mM sodium phosphate buffer, pH 5.3. The sample and 0.06% hyaluronic acid were reacted at 37°C for 45 minutes. After termination of reaction, 40 µl of the enzyme-substrate reaction solution was dispensed into 200 µl of an acidic albumin solution and allowed to stand at room temperature for 19 minutes. Thereafter, the absorbance was measured at 600 nm using a spectrophotometer. The acidic albumin solution is a solution containing 0.1% albumin (BSA) dissolved in 24 mM sodium acetate, 79 mM acetic acid, pH 3.75 buffer. The absorbance value of the measured sample was converted into activity by a standard curve with an activity standard.

When the above process was performed at pH 7.0, the protein sample buffer used was composed of 20 mM sodium phosphate, pH 7.0, 77 mM sodium chloride, and 0.01% (w/v) bovine serum albumin, and the same process was carried out using a 0.06% hyaluronic acid aqueous solution dissolved in 20 mM sodium phosphate buffer, pH 7.0,and 70 mM sodium chloride.

The activity may also be measured in culture medium, but in this case, values equal to or less than 300 units/ml are not reliable, so the LOQ (Limit of Quantification) was set to 300 units/ml, and values equal to or less than this value were indicated as no activity, 0. Also, for activity measurement using purified protein samples, LOQ was set to 15 units/µg.

### [Example 3] Analysis of activity assay results of hyaluronidase variant

The pH-dependent enzyme activities of the native Hyall of SEQ ID NO: 1 and the Hyal1-E131H variant including the amino acid substitution E131H in the native Hyall were investigated.

The results are shown in FIG. 3. FIG. 3A shows results of measuring the enzyme activity of Hyall depending on pH. FIG. 3B shows results of measuring the enzyme activity of the Hyal1-E131H variant depending on pH. It was confirmed that the enzyme activity of Hyall was high at acidic pH, but almost inactive at neutral pH. For the Hyal1-E131H variant, although the enzyme activity was increased at neutral pH compared to acidic pH, it showed relatively very low enzyme activity, so that industrial utility thereof was low.

Based on these results, the design of the variants was expanded to variants near the enzyme active site, and the present invention was carried out by the combination thereof. The characteristics and sequences of novel hyaluronidase Hyall variants according to the present invention are shown in Tables 4 and 5 below.

As shown in Table 4, it was confirmed that the novel hyaluronidase Hyall variants according to the present invention exhibited superior enzyme activity under the conditions of pH 5.3 and pH 7.0, particularly vastly superior enzyme activity when including amino acid substitution of D142K, or further including one or more amino acid substitutions selected from the group consisting of S77D, Q78R, A132E, T143P, Y210H, F212Y, P249N, and V251Q.

**[Table 4]**

| SEQ ID NO: | Code | Amino acid substitution | Culture medium activity (pH 5.3) units/ml | Purified protein activity (pH 5.3) units/µg | Purified protein activity (pH 7.0) units/µg |
|---|---|---|---|---|---|
| 1 | Native | - | <300 (LOQ) | 3.9 | Not done |
| 48 | h2 | S76V | 815 | 2.2 | Not done |
| 49 | h3 | S77D | 1,858 | 3.2 | 3.8 |
| 50 | h4 | Q78R | 1,799 | 2.6 | Not done |
| 51 | h5 | A132E | 2,067 | 3.2 | 3.5 |
| 52 | h6 | D142K | 2,063 | 7.9 | 8.2 |
| 53 | h7 | T143P | 1,240 | 2.2 | Not done |
| 54 | h8 | Y210H | 2,093 | 7.1 | 5.8 |
| 55 | h9 | F212Y | 1,299 | 6.1 | Not done |
| 56 | h10 | L213K | 912 | 4.0 | Not done |
| 57 | h11 | V251Q | 1,722 | 2.8 | Not done |
| 58 | h12 | P249N | 586 | 8.7 | 11.1 |
| 59 | h13 | Q288R | <300 | 2.0 | Not done |
| 60 | h14 | D292T | <300 | 2.0 | Not done |
| 61 | h22 | S77D, D142K, Y210H | 513 | 9 | 5.1 |
| 62 | h23 | S77D, Q78R, D142K, Y210H | 353 | 8.8 | 10.9 |
| 63 | h24 | S77D, A132E, D142K, Y210H | 697 | 7.2 | 11.7 |
| 64 | h25 | S77D, D142K, T143P, Y210H | 619 | 5.8 | Not done |
| 65 | h26 | S77D, D142K, Y210H, F212Y | 428 | 4.6 | Not done |
| 66 | h27 | S77D, D142K, Y210H, V251Q | 683 | 3.8 | Not done |
| 67 | h37 | D142K, Y210H, P249N | <300 | 2.3 | Not done |
| 68 | h38 | D142K, Y210H | 461 | 5.2 | 4.4 |
| 69 | h39 | S77D, A132E | 522 | 4.1 | 4.1 |
| 70 | h40 | S77D, T86D | 472 | 5.3 | 4.6 |

**[Table 5]**

| SEQ ID NO: | Sequence |
|---|---|
| 48 | MAAHLLPICA LFLTLLDMAQ GFRGPLLPNR PFTTVWNANT QWCLERHGVD VDVSVFDVVA NPGQTFRGPD MTIFYVSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN |
| | VTSGALLCSQ ALCSGHGRCV RRTSHPKALL LLNPASFSIQ LTPGGGPLSL RGALSLEDQA QMAVEFKCRC YPGWQAPWCE RKSMW |
| 49 | MAAHLLPICA LFLTLLDMAQ GFRGPLLPNR PFTTVWNANT QWCLERHGVD VDVSVFDVVA NPGQTFRGPD MTIFYSDQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 50 | MAAHLLPICA LFLTLLDMAQ GFRGPLLPNR PFTTVWNANT QWCLERHGVD VDVSVFDVVA NPGQTFRGPD MTIFYSSRLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 51 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EEWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN |
| | VTSGALLCSQ ALCSGHGRCV RRTSHPKALL LLNPASFSIQ LTPGGGPLSL RGALSLEDQA QMAVEFKCRC YPGWQAPWCE RKSMW |
| 52 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WKTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 53 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDPKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 54 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN |
| | VTSGALLCSQ ALCSGHGRCV RRTSHPKALL LLNPASFSIQ LTPGGGPLSL RGALSLEDQA QMAVEFKCRC YPGWQAPWCE RKSMW |
| 55 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DYLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 56 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFKSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 57 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA QLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN |
| | VTSGALLCSQ ALCSGHGRCV RRTSHPKALL LLNPASFSIQ LTPGGGPLSL RGALSLEDQA QMAVEFKCRC YPGWQAPWCE RKSMW |
| 58 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMNA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 59 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVRIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 60 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YTTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 61 | MAAHLLPICA LFLTLLDMAQ GFRGPLLPNR PFTTVWNANT QWCLERHGVD VDVSVFDVVA NPGQTFRGPD MTIFYSDQLG TYPYYTPTGE PVFGGLPQNA SLIAHLARTF QDILAAIPAP DFSGLAVIDW EAWRPRWAFN WKTKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 62 | MAAHLLPICA LFLTLLDMAQ GFRGPLLPNR PFTTVWNANT QWCLERHGVD VDVSVFDVVA NPGQTFRGPD MTIFYSDRLG TYPYYTPTGE PVFGGLPQNA SLIAHLARTF QDILAAIPAP DFSGLAVIDW EAWRPRWAFN WKTKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 63 | MAAHLLPICA LFLTLLDMAQ GFRGPLLPNR PFTTVWNANT QWCLERHGVD VDVSVFDVVA NPGQTFRGPD MTIFYSDQLG TYPYYTPTGE PVFGGLPQNA SLIAHLARTF QDILAAIPAP DFSGLAVIDW EEWRPRWAFN WKTKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 64 | MAAHLLPICA LFLTLLDMAQ GFRGPLLPNR PFTTVWNANT QWCLERHGVD VDVSVFDVVA NPGQTFRGPD MTIFYSDQLG TYPYYTPTGE PVFGGLPQNA SLIAHLARTF QDILAAIPAP DFSGLAVIDW EAWRPRWAFN WKPKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 65 | MAAHLLPICA LFLTLLDMAQ GFRGPLLPNR PFTTVWNANT QWCLERHGVD VDVSVFDVVA NPGQTFRGPD MTIFYSDQLG TYPYYTPTGE PVFGGLPQNA SLIAHLARTF QDILAAIPAP DFSGLAVIDW EAWRPRWAFN WKTKDIYRQR SRALVQAQHP DWPAPQVEAV AQDQFQGAAR AWMAGTLQLG RALRPRGLWG FYGFPDCYNH DYLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 66 | MAAHLLPICA LFLTLLDMAQ GFRGPLLPNR PFTTVWNANT QWCLERHGVD VDVSVFDVVA NPGQTFRGPD MTIFYSDQLG TYPYYTPTGE PVFGGLPQNA SLIAHLARTF QDILAAIPAP DFSGLAVIDW EAWRPRWAFN WKTKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMPA QLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 67 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WKTKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMNA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 68 | MAAHLLPICA LFLTLLDMAQ GFRGPLLPNR PFTTVWNANT QWCLERHGVD VDVSVFDVVA NPGQTFRGPD MTIFYSSQLG TYPYYTPTGE PVFGGLPQNA SLIAHLARTF QDILAAIPAP DFSGLAVIDW EAWRPRWAFN WKTKDIYRQR SRALVQAQHP DWPAPQVEAV AQDQFQGAAR AWMAGTLQLG RALRPRGLWG FYGFPDCYNH DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 69 | MAAHLLPICA LFLTLLDMAQ GFRGPLLPNR PFTTVWNANT QWCLERHGVD VDVSVFDVVA NPGQTFRGPD MTIFYSDQLG TYPYYTPTGE PVFGGLPQNA SLIAHLARTF QDILAAIPAP DFSGLAVIDW EEWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |
| 70 | MAAHLLPICA LFLTLLDMAQ GFRGPLLPNR PFTTVWNANT QWCLERHGVD VDVSVFDVVA NPGQTFRGPD MTIFYSDQLG TYPYYDPTGE PVFGGLPQNA SLIAHLARTF QDILAAIPAP DFSGLAVIDW EAWRPRWAFN WDTKDIYRQR SRALVQAQHP DWPAPQVEAV AQDQFQGAAR AWMAGTLQLG RALRPRGLWG FYGFPDCYNY DFLSPNYTGQ CPSGIRAQND QLGWLWGQSR ALYPSIYMPA VLEGTGKSQM YVQHRVAEAF RVAVAAGDPN LPVLPYVQIF YDTTNHFLPL DELEHSLGES AAQGAAGVVL WVSWENTRTK ESCQAIKEYM DTTLGPFILN VTSGALLCSQ ALCSGHGRCV RRTSHPKALL LLNPASFSIQ LTPGGGPLSL RGALSLEDQA QMAVEFKCRC YPGWQAPWCE RKSMW |

### [Industrial Applicability]

According to the present invention, native hyaluronidase variants have structures similar to mature native PH20 and exhibit effects of increasing protein expression level and enzyme activity at neutral pH.

### [Sequence List Free Text]

An electronic file is attached.

## Claims

1. A novel hyaluronidase variant or fragment thereof, comprising amino acid residue substitution at one or more positions in an enzyme active site, a substrate binding site, and/or adjacent to the sites of a native hyaluronidase, and exhibiting hyaluronidase activity.

2. The hyaluronidase variant or fragment thereof according to claim 1, wherein the native hyaluronidase is a mature human native hyaluronidase.

3. The hyaluronidase variant or fragment thereof according to claim 1, wherein the mature human native hyaluronidase is a form of a human native hyaluronidase from which a signal peptide is deleted.

4. The hyaluronidase variant or fragment thereof according to claim 1, wherein the native hyaluronidase is human native Hyal1.

5. The hyaluronidase variant or fragment thereof according to claim 4, wherein the native hyaluronidase comprises a sequence of SEQ ID NO: 1 or an amino acid sequence of F22 to W435 in the sequence of SEQ ID NO: 1.

6. The hyaluronidase variant or fragment thereof according to any one of claims 1 to 5, wherein the novel hyaluronidase variant exhibits hyaluronidase activity at neutral pH.

7. The hyaluronidase variant or fragment thereof according to claim 1, wherein the enzyme active site is D129 and/or E131 in SEQ ID NO: 1.

8. The hyaluronidase variant or fragment thereof according to claim 1, comprising amino acid substitution at one or more positions selected from the group consisting of Q41, W42, G63, S76, S77, Q78, T81, F93, R108, F110, Q111, A115, A116, A132, F139, D142, T143, R148, V170, Q172, G177, A181, Q188, R191, Y210, F212, L213, S214, N216, T218, Q220, Q228, P249, V251, Q263, A267, Q288, D292, T293, T294, H296, H305, L307, L356, R372, L379, G396, A403, M412, E415, and K417 in an amino acid sequence of SEQ ID NO: 1, or amino acid substitution at one or more amino acid positions corresponding to the amino acid in an amino acid sequence of mature human Hyal1.

9. The hyaluronidase variant or fragment thereof according to claim 8, comprising amino acid substitution at one or more positions selected from the group consisting of S76, S77, Q78, A132, D142, T143, Y210, F212, L213, P249, V251, Q288, and D292, or amino acid substitution at one or more amino acid positions corresponding to the amino acid in the amino acid sequence of mature human Hyal1.

10. The hyaluronidase variant or fragment thereof according to claim 8, comprising amino acid residue substitution at D142 or P249, and optionally further comprising amino acid substitution at one or more positions selected from the group consisting of S77, Q78, D142, A132, T143, Y210, F212, P249, and V251, or at one or more amino acid positions corresponding to the amino acid in the amino acid sequence of mature human Hyal1.

11. The hyaluronidase variant or fragment thereof according to claim 8, comprising one or more amino acid substitutions selected from the group consisting of Q41E, W42F, G63R, S76V, S77D, Q78R, T81Y, F93N, R108K, F110K, Q111K, A115F, A116Y, A132E, F139R, D142K, T143P, R148K, V170K, Q172K, G177K, A181D, Q188K, R191K, Y210H, F212Y, L213K, S214K, N216G, T218N, Q220S, Q228R, P249N, V251Q, Q263R, A267R, Q288R, D292T, T293D, T294Q, H296K, H305Y, L307F, L356K, R372K, L379Y, G396K, A403K, M412F, E415K, and K417Y, or amino acid substitution at one or more of the amino acid positions corresponding to the amino acid in the amino acid sequence of mature human Hyal1.

12. The hyaluronidase variant or fragment thereof according to claim 11, comprising one or more amino acid substitutions selected from the group consisting of S76V, S77D, Q78R, A132E, D142K, T143P, Y210H, F212Y, L213K, V251Q, P249N, Q288R, and D292T, or amino acid substitution at one or more amino acid positions corresponding to the amino acid in the amino acid sequence of mature human Hyal1.

13. The hyaluronidase variant or fragment thereof according to claim 11, comprising amino acid substitution of D142K or P249N, and optionally further comprising one or more amino acid substitutions selected from the group consisting of S77D, Q78R, A132E, D142K, T143P, Y210H, F212Y, P249N, and V251Q, or amino acid substitution at one or more amino acid positions corresponding to the amino acid in the amino acid sequence of mature human Hyal1.

14. A therapeutic composition comprising the hyaluronidase variant or fragment thereof according to any one of claims 1 to 13.

15. A nucleic acid encoding the hyaluronidase variant or fragment thereof according to any one of claims 1 to 13.

16. A recombinant expression vector comprising the nucleic acid according to claim 15.

17. A host cell transformed with the recombinant expression vector according to claim 16.

18. The host cell according to claim 17, wherein the host cell is selected from the group consisting of an animal cell, a plant cell, yeast, *Escherichia coli,* and an insect cell.

19. A method of producing a hyaluronidase variant or fragment thereof, comprising culturing the host cell according to claim 18.
